# EUROPEAN PATENT APPLICATION

(11) **EP 4 477 738 A1**
(43) Date of publication of application: **18.12.2024**
(21) Application number: 22937318.8
(22) Date of filing: 29.12.2022
(51) Int. Cl.: C12N 1/19, C12N 15/81, C12N 15/53, C12N 15/31, C12N 15/62, C12P 7/46, A23L 29/00, C12R 1/645

(54) **ENGINEERED STRAIN OF YEAST HAVING MITOCHONDRION-POSITIONED REDUCTIVE TCA PATHWAY AND EFFICIENTLY PRODUCING SUCCINIC ACID, CONSTRUCTION METHOD THEREFOR AND USE THEREOF**

(30) Priority: 15.04.2022 CN 202210395975
(71) Applicant: Shenghong Holding Group Co., Ltd., Suzhou, Jiangsu 215228 (CN)
(72) Inventor: MIAO, Hangen, Suzhou, Jiangsu 215228 (CN); TANG, Jinkui, Suzhou, Jiangsu 215228 (CN); QI, Qingsheng, Suzhou, Jiangsu 215228 (CN); CUI, Zhiyong, Suzhou, Jiangsu 215228 (CN); HOU, Jin, Suzhou, Jiangsu 215228 (CN); ZHONG, Yutao, Suzhou, Jiangsu 215228 (CN); DENG, Jingyu, Suzhou, Jiangsu 215228 (CN)
(74) Representative: Herrmann, Uwe
(86) International application number: PCT/CN2022/143484
(87) International publication number: WO 2023/197692

(57) **Abstract**

The present invention provides an engineered strain of yeast having mitochondrion-positioned reductive TCA pathway and efficiently producing succinic acid, a construction method therefor and use thereof, and belongs to the technical field of microorganisms and fermentation engineering. According to the present invention, the enzymes related to the biosynthesis pathway of succinic acid in yeast cells are overexpressed and positioned to the mitochondrial matrix to facilitate the functional expression of the reductive TCA pathway in eukaryotic microorganisms. The strain can utilize common carbon and nitrogen sources, and after fed-batch fermentation culture, the succinic acid yield reaches 50.1 g/L, and the conversion rate reaches 0.75 g/g of glucose. As an excellent strain for efficiently producing succinic acid, the strain has good application prospects in the fields of food, chemical engineering, degradable materials, and the like.

## Description

The present invention claims priority to Chinese Patent Application No. 202210395975.3, titled "engineered yeast strain with mitochondrial-targeted reductive TCA pathway for high succinic acid production, construction method therefor and use thereof," filed on April 15, 2022, with the China National Intellectual Property Administration, the entirety of which is incorporated herein by reference.

### TECHNICAL FIELD

The present invention relates to the field of microbiology and fermentation engineering, specifically to an engineered yeast strain with mitochondrial-targeted reductive TCA pathway for high succinic acid production, construction method therefor and use thereof.

### BACKGROUND

The background information disclosed in the present invention is intended only to enhance the understanding of the general background of the invention and should not necessarily be considered as an acknowledgment or any form of implication that the information constitutes prior art that is generally known by those skilled in the art.

Succinic acid (SA), also known as butanedioic acid, is an important C4 compound widely used in detergents, surfactants, food additives, antimicrobial agents, and the pharmaceutical industry. It has been selected by the U.S. Department of Energy as one of the twelve most commercially valuable platform chemicals. Succinic acid is also a crucial raw material for producing biodegradable plastics, such as polybutylene succinate (PBS) and polybutylene succinate adipate (PBSA). The current production capacity of succinic acid cannot meet market demand. While chemical processes are primarily used for the synthesis of succinic acid, they suffer from disadvantages such as high energy consumption, severe pollution, and greenhouse gas emissions compared to the biological fermentation method. Therefore, developing efficient, green, and sustainable fermentation technology for succinic acid production is of significant practical importance.

Compared to bacterial hosts, yeast has higher tolerance to acids, bases and osmotic pressure, allowing for low pH fermentation of succinic acid, which simplifies the fermentation process and reduces downstream product processing costs. In recent years, many researchers have attempted to modify the metabolic pathways of various yeast cells, including *Saccharomyces cerevisiae (S. cerevisiae), Pichia kudriavzevii, Issatchenkia orientalis,* and *Yarrowia lipolytica,* to achieve the fermentation production of succinic acid. *Yarrowia lipolytica,* a non-conventional yeast, has gained increasing attention as a potential strain for succinic acid production due to its high safety, strong acid tolerance, secretion of various metabolic products, ability to utilize a variety of carbohydrates and so on. *Yarrowia lipolytica* can produce succinic acid via the oxidative TCA pathway by reducing succinate dehydrogenase activity. However, the theoretical carbon conversion rate of succinic acid synthesis via the oxidative TCA pathway is only 0.65 g/g glucose, and the actual conversion rate of succinic acid in the current engineered strains of *Yarrowia lipolytica* still lags behind other bacterial production strains, such as *Escherichia coli (E. coli), Corynebacterium glutamicum,* and *Actinobacillus succinogenes.*

Among the known metabolic pathways related to the biosynthesis of succinic acid, the reductive TCA branch has a higher theoretical conversion efficiency (approximately 1.1 g/g glucose) and can significantly reduce greenhouse gas CO₂ emissions, offering distinct advantages over other pathways. Some bacteria isolated from natural environments, such as *Actinobacillus succinogenes* and *Mannheimia succiniciproducens,* can efficiently synthesize succinic acid through their inherent reductive TCA pathway. However, most yeasts lack the key enzyme fumarate reductase, which catalyzes the conversion of fumarate to succinic acid, and the construction and expression of exogenous reductive TCA pathways are relatively challenging. Therefore, constructing a functional reductive TCA pathway in yeast cells and achieving compatibility between exogenous succinic acid synthesis pathways and the host is critical for improving succinic acid production efficiency.

### SUMMARY

In response to the problems present in the prior art, the purpose of the present invention is to provide an engineered yeast strain with mitochondrial-targeted reductive TCA pathway for high succinic acid production, construction method therefor and use thereof. The present invention utilizes the strictly aerobic yeast strain *Yarrowia lipolytica* as a starting strain, targeting different types of fumarate reductases to the mitochondrial matrix. This demonstrates that the subcellular relocalization of these enzymes helps enhance succinic acid production. Using the mitochondrial-targeted NADH-dependent fumarate reductase overexpression strain *Yarrowia lipolytica* Mbmr-SA1 as the host, genes related to succinic acid biosynthesis are overexpressed, ultimately resulting in a high-efficiency succinic acid-producing strain *Yarrowia lipolytica* Mbmr-SA4. This strain has the potential to be utilized as an industrial strain for the production of succinic acid. Based on the above research findings, the present invention is completed.

In the first aspect of the present invention, an engineered yeast strain with a mitochondrial-targeted reductive TCA pathway for high succinic acid production is provided. The engineered yeast strain expresses a fumarate reductase, with the fumarate reductase being overexpressed and relocalized to the mitochondrial matrix, and additionally overexpresses enzymes related to succinic acid synthesis and a dicarboxylate transport protein. The yeast engineering strain possesses a functional reductive succinic acid biosynthesis pathway, enabling efficient production of succinic acid using glucose-containing media.

In the second aspect of the present invention, a construction method for the aforementioned high succinic acid-producing yeast engineering strain with a mitochondrial targeted reductive TCA pathway is provided. The construction method includes:
using a yeast strain as the starting strain, overexpressing mitochondrial-targeted fumarase and endogenous malate dehydrogenase, followed by overexpressing dicarboxylate transport proteins.

In the third aspect of the present invention, an industrial method for producing succinic acid is provided, which includes culturing and fermenting the aforementioned yeast engineering strain.

In the fourth aspect of the present invention, an application of the aforementioned yeast engineering strain or the industrial production method in the fields of chemicals, biodegradable materials, food, and pharmaceuticals is provided.

### Beneficial technical effects of the above technical solutions:

The above technical solutions disclose a new *Yarrowia lipolytica* engineering strain, which can be used for high succinic acid production. As an engineering strain for succinic acid, it can achieve desirable yields and conversion rates. The strain can utilize common carbon and nitrogen sources, reaching a succinic acid yield of 50.1 g/L and a conversion rate of 0.75 g/g glucose after batch-fed fermentation and cultivation. Additionally, studies have shown that the engineered strain can perform low pH fermentation during cultivation, which effectively reduces the need for pH adjustments in the culture medium during industrial production, thereby further reducing costs.

The above technical solutions employ new technical methods in constructing the engineered strain Mbmr-SA4, specifically the application of a mitochondrial targeted reductive TCA pathway in high succinic acid production. This significantly enhances succinic acid yield, making the engineered strain highly valuable and promising for practical applications.

### BRIEF DESCRIPTION OF DRAWINGS

The accompanying drawings, which are included as part of the description of the present invention, provide a further understanding of the invention. The schematic embodiments and descriptions of the present invention are intended for explanatory purposes only and should not be considered as unduly limiting the invention.
FIG. 1: Schematic diagram illustrating the mitochondrial targeting of the reductive TCA synthesis pathway used in examples 2 and 3.
FIG. 2: Verification results of the yeast mitochondrial targeting signal in example 2.
   In this figure, hrGFP represents the green fluorescent protein hrGFP overexpressed in the host strain Po1f, while MLS-mCherry represents the red fluorescent protein mCherry, which contains a 23-amino-acid mitochondrial targeting signal sequence, overexpressed in the host strain Po1f. The fluorescence distribution within the cells of both strains was observed under a fluorescence microscope.

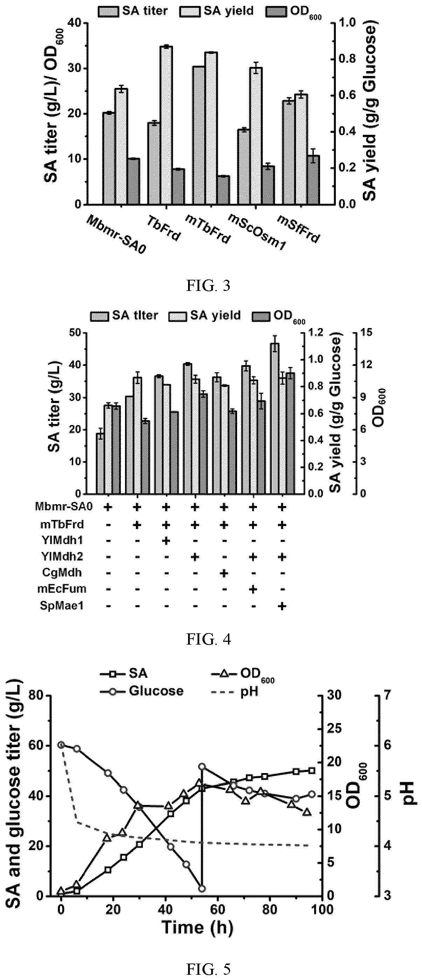

FIG. 3: Results showing the effect of mitochondrial targeting of fumarate reductase on succinic acid production in example 2.
   In this figure, Mbmr-SAO serves as the control strain. TbFrd represents the overexpression of cytosolically localized *Trypanosoma brucei*-derived fumarate reductase in the host strain Mbmr-SAO. mTbFrd represents the overexpression of mitochondrial matrix-targeted *Trypanosoma brucei*-derived fumarate reductase in the host strain Mbmr-SAO. mScOsm1 represents the overexpression of mitochondrial matrix-targeted *Saccharomyces cerevisiae*-derived fumarate reductase in the host strain Mbmr-SAO. mSfFrd represents the overexpression of mitochondrial matrix-targeted *Shewanella frigidimarina-*derived fumarate reductase in the host strain Mbmr-SAO.
FIG. 4: Results showing the effect of overexpression of genes related to the reductive TCA pathway and dicarboxylate transport proteins on succinic acid production in example 3.
   YlMdh1 and YlMdh2 are endogenous malate dehydrogenase-encoding genes, CgMdh is a malate dehydrogenase-encoding gene derived from *Corynebacterium glutamicum,* mEcFum is a mitochondrial-targeted fumarase-encoding gene derived from *Escherichia coli,* and SpMae1 is a dicarboxylate transport protein-encoding gene derived from *Schizosaccharomyces pombe (S. pombe).*
FIG. 5: Succinic acid production yield from shake flask fermentation of the engineered strain Mbmr-SA4 in example 3.

### DETAILED DESCRIPTION

It should be noted that the following detailed description is illustrative and intended to provide a further explanation of the present invention. Unless otherwise indicated, all technical and scientific terms used herein have the same meaning as commonly understood by one of ordinary skill in the art to which this invention belongs.

It should also be noted that the terminology used herein is for the purpose of describing particular embodiments only and is not intended to be limiting of the exemplary embodiments of the present invention. As used herein, unless the context clearly indicates otherwise, the singular forms also include the plural forms, and it is further understood that the terms "includes" and/or "including," when used in this specification, specify the presence of stated features, steps, operations, devices, components, and/or combinations thereof.

In a typical embodiment of the present invention, an engineered yeast strain with a mitochondrial-targeted reductive TCA pathway for high succinic acid production is provided. The engineered yeast strain expresses a fumarate reductase, with the fumarate reductase being overexpressed and relocalized to the mitochondrial matrix, and additionally overexpresses enzymes related to succinic acid synthesis and a dicarboxylate transport protein. This engineered yeast strain has a functional reductive succinic acid biosynthesis pathway and can efficiently produce succinic acid using glucose-containing media.

A starting strain for the engineered yeast strain may include, but is not limited to, *Candida sonorensis, Kluyveromyces marxianus, Kluyveromyces thermotolerana, Issatchenkia orientalis, Candida methanesobosa, Candida lambica, Candida sorboxylosa, Saccharomyces bayanus, Kluyveromyces lactis, Pichia jadinii, Pichia anomala*, *Zygosaccharomyces lentus, Candida zemplinina, Candida geochares, Pichia membranifaciens*, *Saccharomyces cerevisiae,* and *Pichia pastoris.* Preferably, the starting strain is *Yarrowia lipolytica.* More specifically, the starting strain is *Yarrowia lipolytica* strain Mbmr-SA0, with genotype MatA, xpr2-322, axp-2, leu2-270, ura3-302, ΔSdhS::loxP, ΔAch1::loxP, YlPyc, TbFrd, EcFum, YlMdh1, and Pgl ^{G75S}.

The fumarate reductase is an exogenous enzyme, which may be derived from, but is not limited to, *Trypanosoma brucei (T. brucei), Saccharomyces cerevisiae,* and *Shewanella frigidimarina*, preferably from *Trypanosoma brucei.* More specifically, a gene sequence encoding the fumarate reductase is as shown in SEQ ID NO: 1 (see seq_1 in the sequence listing).

The enzymes related to succinic acid synthesis includes, but is not limited to, malate dehydrogenase, and a gene encoding malate dehydrogenase is YlMdh2, with a gene sequence number YALI0E14190p (Gene ID: 2911503).

A gene encoding the dicarboxylate transport protein is SpMae1, derived from *Schizosaccharomyces pombe,* with a nucleotide sequence as shown in SEQ ID NO: 6 (see seq_6 in the sequence listing).

In another embodiment of the present invention, a method for constructing the above high succinic acid-producing yeast engineering strain with a mitochondrial targeted reductive TCA pathway is provided. The construction method includes:
using a yeast strain as a starting strain, overexpressing mitochondrial-targeted fumarase and endogenous malate dehydrogenase, followed by overexpressing dicarboxylate transport proteins.

The yeast strain includes, but is not limited to, *Candida sonorensis, Kluyveromyces marxianus, Kluyveromyces thermotolerana, Issatchenkia orientalis, Candida methanesobosa, Candida lambica, Candida sorboxylosa*, *Saccharomyces bayanus, Kluyveromyces lactis, Pichia jadinii, Pichia anomala, Zygosaccharomyces lentus, Candida zemplinina, Candida geochares, Pichia membranifaciens*, *Saccharomyces cerevisiae,* and *Pichia pastoris.* Preferably, the starting strain is *Yarrowia lipolytica.*

In another embodiment of the present invention, the construction method includes:
using a *Yarrowia lipolytica* strain Mbmr-SAO as the starting strain, introducing an exogenous gene mTbFrd encoding the mitochondrial-targeted fumarase TbFrd into the starting strain, and then sequentially introducing a gene YlMdh2 encoding the malate dehydrogenase and a gene SpMae1 encoding the dicarboxylate transport protein, thereby obtaining a *Yarrowia lipolytica* strain Mbmr-SA4, which is capable of high succinic acid production.

The *Yarrowia lipolytica* strain Mbmr-SAO has genotype comprising MatA, xpr2-322, axp-2, leu2-270, ura3-302, ΔSdhS::loxP, ΔAch1::loxP, YlPyc, TbFrd, EcFum, YlMdh1, and Pgl ^{G75S}; wherein the *Yarrowia lipolytica* strain Mbmr-SAO was constructed from *Yarrowia lipolytica* strain PGC91, which is as described in "Cui, Z., Gao, C., Li, J., Hou, J., Lin, C., & Qi, Q. (2017). Engineering of unconventional yeast Yarrowia lipolytica for efficient succinic acid production from glycerol at low pH. Metabolic engineering, 42, 126-133." and "A method for the aerobic synthesis of succinic acid using a yeast strain *Yarrowia lipolytica* with a reductive TCA pathway." Specifically, a method of constructing the *Yarrowia lipolytica* strain Mbmr-SAO includes: introducing a gene TbFrd (SEQ ID NO: 1), a gene YlMdh1 (sequence number YALI0D16753p, Gene ID: 2910208), and a gene EcFum (SEQ ID NO: 5, see seq_5 in the sequence listing) into *Yarrowia lipolytica* strain PGC91, and mutating the 75th glycine of the 6-phosphogluconolactonase Pgl (sequence number YALI0C19085p, Gene ID: 2909945) to serine.

More specifically, the method involves non-homologous end joining (NHEJ) transformation, in which the linearized plasmids pKi-TbFrd and 113-YlMdh1-EcFum containing the gene expression cassettes of TbFrd, YlMdh1, and EcFum are introduced into the engineered strain *Yarrowia lipolytica* PGC91. Through homologous recombination, the 75th glycine of the 6-phosphogluconolactonase Pgl is mutated to serine, resulting in the high succinic acid-producing recombinant strain Mbmr-SAO.

mTbFrd is obtained by fusing a mitochondrial localization sequence (MLS, SEQ ID NO: 9, see seq_9 in the sequence listing) of the mitochondrial matrix protein cytochrome c oxidase subunit 5b from *Yarrowia lipolytica* with a soluble fumarate reductase TbFrd (NAD-dependent, derived from *T. brucei,* SEQ ID NO: 1), thereby achieving overexpression and relocalization of fumarate reductase to the mitochondrial matrix in the resulting engineered strain. A mitochondrial localization signal peptide (SEQ ID NO: 10, see seq_10 in the sequence listing) encoded by the mitochondrial localization sequence is located at the N-terminus of a fusion protein expressed by the gene mTbFrd.

The malate dehydrogenase-encoding gene is YlMdh2, with the gene sequence number YALI0E14190p (Gene ID: 2911503).

The dicarboxylate transport protein-encoding gene SpMae1 is derived from *Schizosaccharomyces pombe,* and its nucleotide sequence is as shown in SEQ ID NO: 6 (see seq_6 in the sequence listing).

In another embodiment of the present invention, a method for the industrial production of succinic acid is provided, which includes culturing and fermenting the above engineered yeast strain.

Specifically, a method for culturing and fermenting the engineered yeast strain includes fermenting and culturing the above engineered yeast strain in YPD medium.

More specifically, the method for culturing and fermenting the engineered yeast strain includes: controlling a temperature at 25-35°C (preferably 30°C) during the fermentation phase, maintaining an aeration rate of 0.5-5 vvm (preferably 1.0 vvm), and controlling a stirring speed at 100-800 rpm (preferably 500 rpm).

More specifically, the method for culturing and fermenting the engineered yeast strain also includes monitoring glucose concentration in the fermentation broth, and when the glucose concentration falls below 10 g/L, supplementing with glucose to 50-60 g/L. The method does not require pH adjustment, making the operation more convenient and cost-effective.

The YPD medium includes: 2% tryptone, 1% yeast extract, and 6% glucose.

In another embodiment of the present invention, an application of the above engineered yeast strain or the industrial production method in the fields of chemicals, biodegradable materials, food, and pharmaceuticals is provided.

The following examples further illustrate the present invention, but do not limit the invention. It should be understood that these examples are only for illustrating the present invention and are not intended to limit the scope of the present invention.

### Example 1

### I. Materials and Methods

1. The gene synthesis in the present invention was completed by General Biosystems (Anhui) Co., Ltd.; the primer synthesis and sequencing were performed by Tsingke Biotechnology Co., Ltd.
2. The experimental methods used in the following examples, including plasmid construction, enzyme digestion, competent cell preparation, transformation, etc., were conventional methods unless otherwise specified. Specific experimental conditions could be determined through simple experiments if necessary.
3. The materials, reagents, etc., used in the following examples were commercially available unless otherwise specified.
4. The *Yarrowia lipolytica* strain Mbmr-SAO (genotype MatA, xpr2-322, axp-2, leu2-270, ura3-302, ΔSdh5::loxP, ΔAch1 ::loxP, YlPyc, TbFrd, EcFum, YlMdh1, and Pgl ^{G75S}) in the present invention was constructed based on the engineered strain PGC91 (MatA, xpr2-322, axp-2, leu2-270, ura3-302, ΔSdh5::loxP, ΔAch1::loxP, YlPyc, see "Cui, Z., Gao, C., Li, J., Hou, J., Lin, C., & Qi, Q. (2017). Engineering of unconventional yeast Yarrowia lipolytica for efficient succinic acid production from glycerol at low pH. Metabolic engineering, 42, 126-133." and "CN107916275A, a method for the aerobic synthesis of succinic acid using a *Yarrowia lipolytica* strain with a reductive TCA pathway"). It also involved overexpression vectors carrying a recyclable selection marker, pKi-1, pKi-hyg, pKi-ura, 113-GPD-TEF, and JMP-hyg-GPD, the construction steps as described in the literature (Cui, Z., Jiang, X., Zheng, H., Qi, Q., & Hou, J. (2019). Homology-independent genome integration enables rapid library construction for enzyme expression and pathway optimization in Yarrowia lipolytica. Biotechnology and bioengineering, 116(2), 354-363.). The construction of the JMP113 plasmid and site-specific recombinase overexpression vector pUB4-CRE was described in the literature (Fickers, P., Le Dall, M. T., Gaillardin, C., Thonart, P., & Nicaud, J. M. (2003). New disruption cassettes for rapid gene disruption and marker rescue in the yeast Yarrowia lipolytica. Journal of microbiological methods, 55(3), 727-737.).
5. The genes involved in the present invention, including TbFrd from *T. brucei,* SfFrd from *Shewanella frigidimarina,* ScOsm1 from *Saccharomyces cerevisiae,* CgMdh from *Corynebacterium glutamicum,* fumC from *E. coli,* and SpMae from *S. pombe,* were codon-optimized and synthesized by General Biosystems (Chuzhou, China). The YlMdh1 and YlMdh2 genes were cloned from the *Yarrowia lipolytica* genome.
6. LB solid medium: 10 g/L tryptone, 5 g/L yeast extract, 10 g/L sodium chloride, 20 g/L agar.

LB liquid medium: 10 g/L tryptone, 5 g/L yeast extract, 10 g/L sodium chloride.
YPD medium: 10 g/L yeast extract, 20 g/L peptone, 20 g/L glucose.
YPD solid medium: 10 g/L yeast extract, 20 g/L peptone, 20 g/L glucose, 2% agar. SD liquid selection medium: 26.7 g/L SD base medium, supplemented with an appropriate concentration of amino acid dropout mix, adjusted to pH 6.0.
SD solid medium: SD liquid selection medium supplemented with 2% agar.

### II. Amplification of Genetic Elements and Preparation of Target Plasmids

### (i) Preparation of Target Genes

1. The nucleotide sequence of the NADH-dependent fumarate reductase gene TbFrd from *T. brucei* (accession number KT026107.1) provided by NCBI was codon-optimized and synthesized by General Biosystems (Anhui) Co., Ltd. The nucleotide sequence of the optimized NADH-dependent fumarate reductase gene TbFrdY is shown in SEQ ID NO: 1.
2. The nucleotide sequence of the FADH₂-dependent fumarate reductase gene ScOsm1 (accession number YJR051W) from *S. cerevisiae* provided by NCBI was codon-optimized and synthesized by General Biosystems (Anhui) Co., Ltd. The nucleotide sequence of the optimized FADH₂-dependent fumarate reductase gene ScOsm1Y is shown in SEQ ID NO: 2 (see seq_2 in the sequence listing).
3. The nucleotide sequence of the electron transport-dependent fumarate reductase gene SfFrd (GenBank: L04283.1) from *Shewanella frigidimarina* provided by NCBI was codon-optimized and synthesized by General Biosystems (Anhui) Co., Ltd. The nucleotide sequence of the optimized fumarate reductase gene SfFrdY is shown in SEQ ID NO: 3 (see seq_3 in the sequence listing).
4. The nucleotide sequence of the malate dehydrogenase-encoding gene CgMdh (accession number Cgl2380) from *C. glutamicum* provided by NCBI was codon-optimized and synthesized by General Biosystems (Anhui) Co., Ltd. The nucleotide sequence of the optimized malate dehydrogenase-encoding gene CgMdhY is shown in SEQ ID NO: 4 (see seq_4 in the sequence listing).
5. The nucleotide sequence of the fumarase-encoding gene EcFum (accession number b1611) from *E. coli* provided by NCBI was codon-optimized and synthesized by General Biosystems (Anhui) Co., Ltd. The nucleotide sequence of the optimized fumarase-encoding gene EcFumY is shown in SEQ ID NO: 5.
6. The nucleotide sequence of the C4 dicarboxylate transport protein-encoding gene SpMae (accession number SPAPB8E5.03) from *S. pombe* provided by NCBI was codon-optimized and synthesized by General Biosystems (Anhui) Co., Ltd. The nucleotide sequence of the optimized C4 dicarboxylate transport protein-encoding gene SpMaeY is shown in SEQ ID NO: 6.
7. The nucleotide sequences of the malate dehydrogenase genes YlMdh1 (accession number YALI0D16753p, Gene ID: 2910208) and YlMdh2 (accession number YALI0E14190p) as well as the 6-phosphogluconolactonase gene Pgl (accession number YALI0C19085p, Gene ID: 2909945) provided by NCBI were obtained from the *Yarrowia lipolytica* genome by PCR.

### (ii) Plasmid Construction

### 1. Construction of plasmid pKi-TbFrd

Based on the codon-optimized TbFrdY gene sequence (SEQ ID NO: 1) and the expression plasmid pKi-1 sequence, primers were designed as follows:
TbFrd-F:
   ATAAGAATCATTCAAAGGTTATGGTGGACGGTCGATCTTC.
TbFrd-R:
   ACATAACTAATTACATGATTTTAGGAGCCAGAGGGCTCGG.

Using the codon-optimized synthesized TbFrdY gene sequence as a template, the assembly fragment with corresponding terminal homologous sequences was obtained by PCR (polymerase chain reaction) amplification with primers TbFrd-F and TbFrd-R. The PCR reaction conditions were as follows: 97°C pre-denaturation for 5 min, 94°C denaturation for 60 s, 56°C annealing for 30 s, 72°C extension for 3 min, followed by 30 cycles of 72°C extension for 10 min, and storage at 4°C.

The pKi-1 plasmid was digested with the Bsp119 I restriction enzyme, followed by recovery and purification. The digested fragment and PCR product were ligated using a Gibson Assembly Cloning Kit (New England Biolabs (NEB), England) to construct the pKi-TbFrd plasmid.

### 2. Construction of plasmid 113-YlMdh1-EcFum

Based on the codon-optimized EcFumY gene sequence (SEQ ID NO: 5), the YlMdh1 gene sequence (YALI0D16753p), and the expression plasmid 113-GPD-TEF sequence, primers were designed as follows:
YlMdh1-F:
   CAGTACTAACCGCAGATTTATGTTCCGAACCCGAGTTAC.
YlMdh1-R:
   TAACTAATTACATGAATTTTTAAGGGTTCTGCTTGACAA.
EcFum-F:
   ATTAAACACACATCAACAGATGATGAACACCGTGCGATC.
EcFum-R:
   GACAGGCCATGGAGGTACGTTATCGTCCGGCCTTCATAG.

Using the *Yarrowia lipolytica* W29 genome and the codon-optimized synthesized EcFumY gene sequence as templates, assembly fragments with corresponding terminal homologous sequences were obtained by PCR amplification with primers YlMdh1-F, YlMdh1-R, EcFum-F and EcFum-R.

The 113-GPD-TEF plasmid was digested with SmiI and SalI restriction enzymes, followed by recovery and purification. The digested fragment and PCR product were ligated using Gibson Assembly to construct the 113-YlMdh1-EcFum plasmid.

### 3. Construction of the Pgl ^{G75S} mutant fragment

Based on the *Yarrowia lipolytica* 6-phosphogluconolactonase-encoding gene Pgl (YALI0C19085p) and its upstream and downstream sequences from NCBI, as well as the plasmid JMP113 sequence, primers were designed as follows:
Pgl-UP-F:
   AATTTATCTCATTGTCACTCAAT.
Pgl-UP-R:
   TATTTCGCGTCTCGGCCTCT.
Pgl-URA3-F:
   AGGCCGAGACGCGAAATAGATAACAGGGTAATTATCGCTTCG.
Pgl-URA3-R:
   TTGCTTGGTGGAGTCTGGGGATGATTACCCTGTTATCCCTACA.
Pgl-DN-F:
   CCCAGACTCCACCAAGCAA.
Pgl^{G75S}-R:
   TCAGCGAGCCGACGGAAATGCCGATTCGGAA.
Pgl^{G75S}-F:
   GGCATTTCCGGCGTCTCGCTGATCCACGTGC.
Pgl-DN-R:
   GTGATTTTGAAGTCTTTTACTTGTG.

Using the *Yarrowia lipolytica* W29 genome and the JMP113 plasmid as templates, fusion fragments with corresponding terminal overlapping sequences were obtained by PCR amplification with the above primers. Subsequently, the Pgl ^{G75S} fragment, in which the 75th glycine was mutated to serine, was obtained by overlap extension PCR.

### 4. Construction of plasmid pKi-mTbFrd

Based on the codon-optimized TbFrdY gene sequence (SEQ ID NO: 1), the cytochrome c oxidase subunit 5b mitochondrial localization sequence (SEQ ID NO: 9), and the expression plasmid pKi-1 sequence, primers were designed as follows:
UT8-MLS-F:
   TAAGAATCATTCAAAGGTTATGTTCGCCCTGAGAAGATC.
MLS-R:
   GAATCGAGCAACCTGCTGGG.
mTbFrd-F:
   CCAGCAGGTTGCTCGATTCGTGGACGGTCGATCTTCCGC.
TbFrd-R:
   ACATAACTAATTACATGATTTTAGGAGCCAGAGGGCTCGG.

Using the *Yarrowia lipolytica* W29 genome and the codon-optimized synthesized TbFrdY gene sequence as templates, fusion fragments with corresponding terminal overlapping sequences were obtained by PCR amplification with primers UT8-MLS-F, MLS-R, mTbFrd-F and TbFrd-R. Subsequently, assembly fragments were obtained by overlap extension PCR.

The pKi-1 plasmid was digested with the Bsp119 I restriction enzyme, followed by recovery and purification. The digested fragment and PCR product were ligated using Gibson Assembly to construct the pKi-mTbFrd plasmid.

### 5. Construction of plasmid pKi-mScOsm1

Based on the codon-optimized ScOsm1Y gene sequence (SEQ ID NO: 2), the cytochrome c oxidase subunit 5b mitochondrial localization sequence (MFALRRSLLSAGRIARPQQVARF, SEQ ID NO: 9), and the expression plasmid pKi-1 sequence, primers were designed as follows:
UT8-MLS-F:
   TAAGAATCATTCAAAGGTTATGTTCGCCCTGAGAAGATC.
MLS-R:
   GAATCGAGCAACCTGCTGGG.
mScOsm1-F:
   CAGCAGGTTGCTCGATTCTTCGCCCTGCGACGATCTCT.
mScOsm1-R:
   ATAACTAATTACATGATTTTAGTACAGCTTGGCAATGT.

Using the *Yarrowia lipolytica* W29 genome and the codon-optimized synthesized ScOsm1Y gene sequence as templates, fusion fragments with corresponding terminal overlapping sequences were obtained by PCR amplification with primers UT8-MLS-F, MLS-R, mScOsm1-F and mScOsm1-R. Subsequently, assembly fragments were obtained by overlap extension PCR.

The pKi-1 plasmid was digested with the Bsp119 I restriction enzyme, followed by recovery and purification. The digested fragment and PCR product were ligated using Gibson Assembly to construct the pKi-mScOsm1 plasmid.

### 6. Construction of plasmid pKi-mSfFrd

Based on the codon-optimized SfFrdY gene sequence (SEQ ID NO: 3), the cytochrome c oxidase subunit 5b mitochondrial localization sequence (SEQ ID NO: 9), and the expression plasmid pKi-1 sequence, primers were designed as follows:
UT8-MLS-F:
   TAAGAATCATTCAAAGGTTATGTTCGCCCTGAGAAGATC.
MLS-R:
   GAATCGAGCAACCTGCTGGG.
m SfFrd-F:
   CCAGCAGGTTGCTCGATTCAAGAAGATGAACCTGGCCGT.
mSfFrd-R:
   CATAACTAATTACATGATTTTAGTTCTTCTTAGAGTACT.

Using the *Yarrowia lipolytica* W29 genome and the codon-optimized synthesized SfFrdY gene sequence as templates, fusion fragments with corresponding terminal overlapping sequences were obtained by PCR amplification with primers UT8-MLS-F, MLS-R, mSfFrd-F and mSfFrd-R. Subsequently, assembly fragments were obtained by overlap extension PCR.

The pKi-1 plasmid was digested with the Bsp119 I restriction enzyme, followed by recovery and purification. The digested fragment and PCR product were ligated using Gibson Assembly to construct the pKi-mSfFrd plasmid.

### 7. Construction of plasmid 113-GPD-YlMdh1

Based on the YlMdh1 gene sequence (YALI0D16753p) from NCBI, and the expression plasmid 113-GPD-TEF sequence, primers were designed as follows:
YlMdh1-F:
   ATTAAACACACATCAACAGATGTTCCGAACCCGAGTTAC.
YlMdh1-R:
   GACAGGCCATGGAGGTACGTTAAGGGTTCTGCTTGACAA.

Using the *Yarrowia lipolytica* W29 genome as a template, the assembly fragment with corresponding terminal homologous sequences was obtained by PCR amplification with primers YlMdh1-F and YlMdh1-R.

The 113-GPD-TEF plasmid was digested with the SalI restriction enzyme, followed by recovery and purification. The digested fragment and PCR product were ligated using a Gibson Assembly Cloning Kit (New England Biolabs (NEB), England) to construct the 113-GPD-YlMdh1 plasmid.

### 8. Construction of plasmid 113-GPD-YlMdh2

Based on the YlMdh2 gene sequence (YALI0E14190p) from NCBI, and the expression plasmid 113-GPD-TEF sequence, primers were designed as follows:
YlMdh2-F:
   ATTAAACACACATCAACAGATGGTTAAAGCTGTCGTTGC.
YlMdh2-R:
   GACAGGCCATGGAGGTACGCTAGTTGGCAGGAGGAGGGT.

Using the *Yarrowia lipolytica* W29 genome as a template, the assembly fragment with corresponding terminal homologous sequences was obtained by PCR amplification with primers YlMdh2-F and YlMdh2-R.

The 113-GPD-TEF plasmid was digested with the SalI restriction enzyme, followed by recovery and purification. The digested fragment and PCR product were ligated using a Gibson Assembly Cloning Kit (New England Biolabs (NEB), England) to construct the 113-GPD-YlMdh2 plasmid.

### 9. Construction of plasmid 113-GPD-CgMdh

Based on the codon-optimized CgMdh gene sequence (SEQ ID NO: 4) and the expression plasmid 113-GPD-TEF sequence, primers were designed as follows:
CgMdh-F:
   ATTAAACACACATCAACAGATGAACTCTCCCCAGAACGT.
CgMdh-R:
   GACAGGCCATGGAGGTACGTTACAGCAGATCTCGCACGG.

Using the *Yarrowia lipolytica* W29 genome as a template, the assembly fragment with corresponding terminal homologous sequences was obtained by PCR amplification with primers CgMdh-F and CgMdh-R.

The 113-GPD-TEF plasmid was digested with the SalI restriction enzyme, followed by recovery and purification. The digested fragment and PCR product were ligated using a Gibson Assembly Cloning Kit (New England Biolabs (NEB), England) to construct the 113-GPD-CgMdh plasmid.

### 10. Construction of plasmid 113-YlMdh2-mEcFum

Based on the codon-optimized EcFumY gene sequence (SEQ ID NO: 5), the cytochrome c oxidase subunit 5b mitochondrial localization sequence (SEQ ID NO: 9), and the expression plasmid 113-GPD-YlMdh2 sequence, primers were designed as follows:
pTEFin-MI,S-F:
   TTTGCAGTACTAACCGCAGTTCGCCCTGAGAAGATCTCT.
MLS-R:
   GAATCGAGCAACCTGCTGGG.
mEcFum-F:
   CCCAGCAGGTTGCTCGATTCATGAACACCGTGCGATCTG.
mEcFum-R:
   CATAACTAATTACATGAATTTATCGTCCGGCCTTCATAG.

Using the *Yarrowia lipolytica* W29 genome and the codon-optimized synthesized SfFrdY gene sequence as templates, fusion fragments with corresponding terminal overlapping sequences were obtained by PCR amplification with primers UT8-MLS-F, MLS-R, mEcFum-F and mEcFum-R. Subsequently, assembly fragments were obtained by overlap extension PCR.

The 113-GPD-YlMdh2 plasmid was digested with the SmiI restriction enzyme, followed by recovery and purification. The digested fragment and PCR product were ligated using a Gibson Assembly Cloning Kit (New England Biolabs (NEB), England) to construct the 113-YlMdh2-mEcFum plasmid.

### 11. Construction of plasmid pKi-hyg-SpMae

Based on the codon-optimized SpMaeY gene sequence (SEQ ID NO: 6) and the expression plasmid pKi-hyg sequence, primers were designed as follows:
SpMae-F:
   TAAGAATCATTCAAAGGTTATGGGCGAGCTGAAGGAAAT.
SpMae-R:
   ACATAACTAATTACATGATTTTAGGAGCCAGAGGGCTCGG.

Using the codon-optimized synthesized SpMaeY gene sequence as a template, the assembly fragment with corresponding terminal homologous sequences was obtained by PCR amplification with primers SpMae-F and SpMae-R.

The pKi-hyg plasmid was digested with the Bsp119 I restriction enzyme, followed by recovery and purification. The digested fragment and PCR product were ligated using Gibson Assembly to construct the pKi-hyg-SpMae plasmid.

### 12. Construction of plasmid YLEP-hrGFP

Based on the codon-optimized hrGFP gene sequence (SEQ ID NO: 7, see seq_7 in the sequence listing) and the free expression vector YLEP-leu sequence, primers were designed as follows:
hrGFP-F:
   TAAGAATCATTCAAAGGTTATGGTGAGCAAGCAGATCCT.
hrGFP-R:
   CATAACTAATTACATGATTTTACACCCACTCGTGCAGGC.

Using the codon-optimized synthesized hrGFP gene sequence as a template, the assembly fragment with corresponding terminal homologous sequences was obtained by PCR amplification with primers hrGFP-F and hrGFP-R.

The YLEP-leu plasmid was digested with the Bsp119 I restriction enzyme, followed by recovery and purification. The digested fragment and PCR product were ligated using Gibson Assembly to construct the YLEP-hrGFP plasmid.

### 13. Construction of plasmid YLEP-MLS-mCherry

Based on the codon-optimized mCherry gene sequence (SEQ ID NO: 8, see seq_8 in the sequence listing), the cytochrome c oxidase subunit 5b mitochondrial localization sequence (SEQ ID NO: 9), and the free expression vector YLEP-leu sequence, primers were designed as follows:
UT8-MLS-F:
   TAAGAATCATTCAAAGGTTATGTTCGCCCTGAGAAGATC.
MLS-R:
   GAATCGAGCAACCTGCTGGG.
mCherry-F:
   ACTGCACACGACCTCGCTGGTGAGCAAGGGCGAGGAGGA.
mCherry-R:
   CATAACTAATTACATGATTCTACTTGTACAGCTCGTCCA.

Using the *Yarrowia lipolytica* W29 genome and the codon-optimized synthesized mCherry gene sequence as templates, fusion fragments with corresponding terminal overlapping sequences were obtained by PCR amplification with primers UT8-MLS-F, MLS-R, mCherry-F and mCherry-R. Subsequently, assembly fragments were obtained by overlap extension PCR.

The YLEP-leu plasmid was digested with the Bsp119 I restriction enzyme, followed by recovery and purification. The digested fragment and PCR product were ligated using Gibson Assembly to construct the YLEP-MLS-mCherry plasmid.

### Example 2: Mitochondrial Targeting of Fumarate Reductase and Its Effect on Succinic Acid Synthesis

In this example, the non-homologous end joining (NHEJ) transformation method was used to introduce the linearized plasmids pKi-TbFrd and 113-YlMdh1-EcFum, containing the gene expression cassettes of TbFrd, YlMdh1, and EcFum, into the engineered strain *Yarrowia lipolytica* PGC91. Through homologous recombination, the 75th glycine of the 6-phosphogluconolactonase Pgl was mutated to serine, resulting in the high succinic acid-producing recombinant strain Mbmr-SAO.

The specific method was as follows: (1) *Yarrowia lipolytica* PGC91 was cultured overnight in YPG liquid medium (containing 2% peptone, 1% yeast extract, and 2% glycerol), and competent cells were prepared using the conventional yeast lithium acetate method. (2) To 40 µL of competent cells, 2 µL of pKi-TbFrd and 113-YlMdh1-EcFum plasmid fragments were added, along with 2 µL of salmon sperm DNA, and the mixture was incubated in a water bath at 30°C for 15 minutes. (3) To the above mixture, 350 µL of PEG 4000-Lithium Acetate (0.1 M, pH 6.0) and 16 µL of 1MDTT (40 mM) were added, and the mixture was incubated in a water bath at 30°C without shaking for 1 hour. (4) Then, 40 µL of DMSO (final concentration approximately 40%) was added, and the mixture was heat-shocked at 39°C for 10 minutes. (5) 600 µL of Lithium Acetate (0.1 M, pH 6.0) was added, and the mixture was left at room temperature for 15 minutes. (6) 200 µL of the above mixture was spread on SD selection plates and incubated at 30°C for 2-3 days. (6) Random transformants were selected, subjected to shake flask fermentation, and the succinic acid in the fermentation broth was detected.

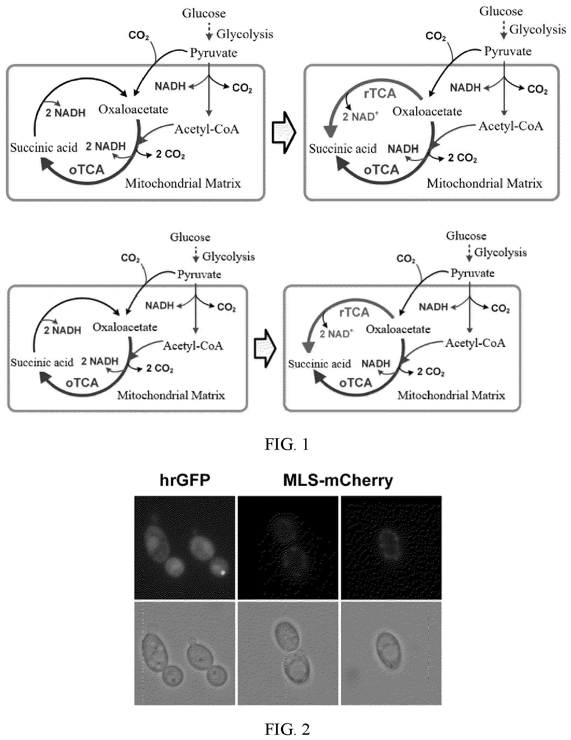

The fermentation and detection methods for succinic acid were as follows: single colonies grown on the plates were inoculated into 50 mL flasks containing 10 mL of YPG liquid medium (2% peptone, 1% yeast extract, 2% glycerol) and cultured with shaking at 30°C and 220 rpm for activation. Then, 1 mL of the activated culture was transferred to 250 mL flasks containing 50 mL of fermentation medium (2% peptone, 1% yeast extract, 6% glucose) and cultured at 30°C and 120 rpm for 96 hours. No external acid or alkali was added to maintain the neutral pH of the fermentation broth. During fermentation, samples were taken every 12-24 hours, and the optical absorption value of the culture was measured at 600 nm. Specifically, 1 mL of the culture was centrifuged at 10,000-12,000 rpm for 2 minutes, the supernatant was discarded, and the cells were resuspended in an equal volume of water. After appropriate dilution, the optical absorption value was measured using a spectrophotometer. Metabolites such as carbon sources and organic acids in the fermentation broth were analyzed by high-performance liquid chromatography (HPLC). Specifically, 1 mL of the fermentation broth was centrifuged at 12,000 rpm at room temperature for 2 minutes, the supernatant was filtered through a 0.22 µm microporous membrane, and the organic acid and glucose concentrations were detected using HPLC. The detection conditions were as follows: the column was HPX-87H (BioRad Labs, 300 mm×7.8 mm), the detector was RID-10A, the column temperature was 65°C, the mobile phase was 5 mM H₂SO₄ solution, and the flow rate was 0.6 mL/min. (7) Transformants with high succinic acid yield and conversion rate were selected, and the pUB4-CRE plasmid was introduced into these high-yield strains. The CRE recombinase was constitutively expressed by culturing in YPG-hyg medium at 30°C for 1-2 days. The recombinant clones were streaked on YPG-hyg selection plates, and single colonies were simultaneously transferred to SD selection plates and YPG-hyg plates for cultivation. Clones that grew on the YPG-hyg plates but not on the SD plates indicated that the selection marker genes had been excised by the CRE recombinase. The positive recombinants were further passaged in YPG medium for 2-3 days to select engineered strains which remove the pUB4-CRE plasmid. (8) The Pgl ^{G75S} mutant fragment was transformed into the above succinic acid high-producing strains with the selection marker removed, and the transformants were plated on SD selection plates. Positive transformants were selected and verified using primers F: 5'-ACGACTCAACAAGACACAAAG-3' and R: 5'-ACATGATGGACTCTTCATTG-3'.
2. The mitochondrial localization sequence (MLS) of the mitochondrial matrix protein cytochrome c oxidase subunit 5b from *Yarrowia lipolytica* was identified using the MITOPROT online prediction tool (https://ihg.gsf.de/ihg/mitoprot.html). To verify the function of the MLS sequence, the 23-amino acid targeting sequence (MFALRRSLLSAGRIARPQQVARF) was fused to the N-terminus of the red fluorescent protein mCherry, transformed into the wild-type strain *Yarrowia lipolytica* Po1f, and the intracellular fluorescence distribution was analyzed.
The specific method was as follows: (1) *Yarrowia lipolytica* Po1f was cultured overnight in YPD liquid medium (containing 2% peptone, 1% yeast extract, and 2% glucose), and competent cells were prepared using the conventional yeast lithium acetate method. (2) The YLEP-hrGFP and YLEP-MLS-mCherry plasmids were transformed into Po1f competent cells, spread on SD selection plates, and incubated at 30°C for 2-3 days. (3) Random transformants were selected and cultured in 24-well deep well plates containing corresponding SD selection liquid medium for 24 hours, and fluorescence expression intensity was detected. (4) Cultures with high fluorescence intensity were selected for fluorescence microscopy. As shown in FIG. 2, the mCherry carrying the mitochondrial targeting signal was well expressed, and the fluorescence distribution was relatively concentrated, consistent with the mitochondrial localization characteristics. In contrast, the control green fluorescent protein hrGFP showed a uniform distribution. This indicated that the N-terminal MLS signal sequence could target the desired protein to the mitochondrial matrix.
3. The mitochondrial targeting signal MLS was added to three different types of soluble fumarate reductases: TbFrd (NAD-dependent, derived from *T. brucei*)*,* ScOsm1 (FAD-dependent, derived from *Saccharomyces cerevisiae*)*,* and SfFrd (electron transport-dependent, derived from *Shewanella frigidimarina*)*.* The linearized pKi-mTbFrd, pKi-mScOsm1, and pKi-mSfFrd plasmids were introduced into the Mbmr-SAO strain using the NHEJ transformation method, and the succinic acid synthesis and cell growth were compared. As shown in FIG. 3, the mitochondrial targeting of TbFrd significantly enhanced the succinic acid synthesis capability of the *Yarrowia lipolytica* strain Mbmr-SA1, with succinic acid yield and conversion rate increasing to 30.4 g/L and 0.84 g/g glucose, respectively. The succinic acid conversion rate of the mScOsm1 overexpression strain increased to 0.75 g/g glucose, but the succinic acid yield decreased by 18.3% compared to the control. Although the overexpression of mSfFrd increased the succinic acid yield to 22.8 g/L, the succinic acid conversion rate decreased. These results indicated that TbFrd from *T. brucei* could utilize NADH generated by the mitochondrial oxidative TCA cycle for succinic acid synthesis.

### Example 3: Combined Overexpression of Genes Related to the Reductive TCA Pathway

To further increase the succinic acid production of the strain, this example integrated the linearized plasmids 113-GPD-YlMdh1, 113-GPD-YlMdh2, and 113-GPD-CgMdh into the mTbFRD overexpression strain Mbmr-SA1. The results of YPD shake flask fermentation are shown in FIG. 4. The results indicated that the individual overexpression of the three malate dehydrogenases increased the succinic acid production of Mbmr-SA1, with the highest increase observed in the Mbmr-SA2 strain overexpressing the YlMdh2 gene, resulting in a 32.9% increase in succinic acid production, reaching approximately 40.4 g/L. The present invention further overexpressed the mitochondrial-targeted fumarase-encoding gene mEcFum in the engineered strain Mbmr-SA2 to obtain the strain Mbmr-SA3. The transformants were selected for shake flask fermentation, and the results, as shown in FIG. 4, indicated that the succinic acid yield and conversion rate of the Mbmr-SA3 strain decreased compared to the control, reaching 39.8 g/L and 0.84 g/g glucose, respectively.

Enhancing the extracellular secretion of the final product can reduce the cytotoxicity caused by its excessive accumulation and help alleviate feedback inhibition in the metabolic pathway. Therefore, the present invention further overexpressed the dicarboxylate transport protein SpMae1 on the basis of the Mbmr-SA3 strain. As shown in FIG. 4, the succinic acid yield, conversion rate, and OD₆₀₀ of the SpMae1 overexpression strain Mbmr-SA4 reached the highest levels, approximately 46.7 g/L, 0.86 g/g glucose, and 11.3, respectively. The succinic acid yield and conversion rate reached the highest reported levels for shake flasks, demonstrating potential for industrial application.

### Example 4: Fed-Batch Fermentation of Strain Mbmr-SA4

The high succinic acid-producing *Yarrowia lipolytica* strain Mbmr-SA4, genetically engineered as described in Example 2, was used in a fed-batch fermentation experiment in a 1L fermenter. The medium used in this example was YPD medium (2% tryptone, 1% yeast extract, 6% glucose), with an initial volume of 0.6L. The strain was streaked from a glycerol stock onto YPDG solid plates and incubated for 36 hours. A single colony was then inoculated into a 50 mL YPDG medium shake flask and cultured at 30°C and 220 rpm for 24 hours, which was then used to inoculate the fermenter. The fermenter was set to a temperature of 30°C, an aeration rate of 1.0 vvm, and a stirring speed of 500 rpm, without pH adjustment. Samples were taken every 6 hours to measure glucose concentration. When the glucose concentration dropped below 10 g/L, it was replenished to 60 g/L. Samples were also taken to measure succinic acid and cell biomass. The fermentation results are shown in FIG. 5. Compared to shake flask fermentation, the production rate of succinic acid was higher during the first 54 hours, with a productivity of 0.8 g/h/L. At 96 hours of fermentation, the cell OD₆₀₀ was 12.5, with a succinic acid yield and conversion rate of 50.1 g/L and 0.75 g/g glucose, respectively.

The unspecified matters in the present invention are well-known techniques.

The above descriptions are only preferred embodiments of the present invention and are not intended to limit the present invention. Various modifications and changes can be made by those skilled in the art. Any modifications, equivalent substitutions, or improvements made within the spirit and principles of the present invention should be included within the protection scope of the present invention.

## Claims

1. An engineered yeast strain with a mitochondrial-targeted reductive TCA pathway for high succinic acid production, **characterized in that** the engineered yeast strain expresses a fumarate reductase, with the fumarate reductase being overexpressed and relocalized to the mitochondrial matrix, and additionally overexpresses enzymes related to succinic acid synthesis and a dicarboxylate transport protein.

2. The engineered yeast strain according to claim 1, **characterized in that** a starting strain for the engineered yeast strain comprises *Candida sonorensis, Kluyveromyces marxianus, Kluyveromyces thermotolerana, Issatchenkia orientalis, Candida methanesobosa, Candida lambica, Candida sorboxylosa, Saccharomyces bayanus, Kluyveromyces lactis, Pichia jadinii, Pichia anomala*, *Zygosaccharomyces lentus, Candida zemplinina, Candida geochares, Pichia membranifaciens*, *Saccharomyces cerevisiae, Pichia pastoris;* preferably, the starting strain is *Yarrowia lipolytica;* more preferably, the starting strain is *Yarrowia lipolytica* strain Mbmr-SA0, with genotype MatA, xpr2-322, axp-2, leu2-270, ura3-302, ΔSdh5::loxP, ΔAch1 ::loxP, YlPyc, TbFrd, EcFum, YlMdh1, and Pgl ^{G75S}.

3. The engineered yeast strain according to claim 1, **characterized in that** the fumarate reductase is an exogenous enzyme derived from *Trypanosoma brucei, Saccharomyces cerevisiae,* and *Shewanella frigidimarina,* preferably *Trypanosoma brucei;* more preferably, a gene sequence encoding the fumarate reductase derived from *Trypanosoma brucei* is as shown in SEQ ID NO: 1.

4. The engineered yeast strain according to claim 1, **characterized in that** the enzymes related to succinic acid synthesis comprise malate dehydrogenase, with a malate dehydrogenase-encoding gene being YlMdh2, having a gene number YALI0E14190p.

5. The engineered yeast strain according to claim 1, **characterized in that** a gene encoding the dicarboxylate transport protein is SpMae1, derived from *Schizosaccharomyces pombe,* with a nucleotide sequence as shown in SEQ ID NO: 6.

6. A method for constructing an engineered yeast strain with a mitochondrial-targeted reductive TCA pathway for high succinic acid production as claimed in any one of claims 1-5, **characterized in that** the method comprises:
using a yeast strain as a starting strain, overexpressing mitochondrial-targeted fumarase and endogenous malate dehydrogenase, followed by overexpressing dicarboxylate transport proteins;
preferably, the yeast strain comprises *Candida sonorensis, Kluyveromyces marxianus, Kluyveromyces thermotolerana, Issatchenkia orientalis, Candida methanesobosa, Candida lambica, Candida sorboxylosa, Saccharomyces bayanus, Kluyveromyces lactis, Pichia jadinii, Pichia anomala*, *Zygosaccharomyces lentus, Candida zemplinina, Candida geochares, Pichia membranifaciens*, *Saccharomyces cerevisiae, Pichia pastoris;* further preferably, the starting strain is *Yarrowia lipolytica.*

7. The method according to claim 6, **characterized in that** the method comprises:
using a *Yarrowia lipolytica* strain Mbmr-SAO as the starting strain, introducing an exogenous gene mTbFrd encoding the mitochondrial-targeted fumarase TbFrd into the starting strain, and then sequentially introducing a gene YlMdh2 encoding the malate dehydrogenase and a gene SpMae1 encoding the dicarboxylate transport protein, thereby obtaining a *Yarrowia lipolytica* strain Mbmr-SA4;
preferably, the *Yarrowia lipolytica* strain Mbmr-SAO has genotype comprising MatA, xpr2-322, axp-2, leu2-270, ura3-302, ΔSdhS::loxP, ΔAch1::loxP, YlPyc, TbFrd, EcFum, YlMdh1, and Pgl ^{G75S}; wherein the *Yarrowia lipolytica* strain Mbmr-SAO was constructed from a *Yarrowia lipolytica* strain PGC91, which has genotype comprising MatA, xpr2-322, axp-2, leu2-270, ura3-302, ΔSdhS::loxP, ΔAch1::loxP, and YlPyc through genetic engineering techniques, more preferably, a method of constructing the *Yarrowia lipolytica* strain Mbmr-SAO comprises: introducing a gene TbFrd, with a sequence as shown in SEQ ID NO: 1, a gene YlMdh1, with a sequence number of YALI0D16753p and gene ID of 2910208, and a gene EcFum, with a sequence as shown in SEQ ID NO: 5, into the *Yarrowia lipolytica* strain PGC91, and mutating the 75th glycine of a 6-phosphogluconolactonase Pgl, with a sequence number of YALI0C19085p and gene ID of 2909945 to serine;
preferably, a sequence of the gene mTbFrd is obtained by fusing a mitochondrial localization sequence of a mitochondrial matrix protein cytochrome c oxidase subunit 5b from *Yarrowia lipolytica* having a sequence as shown in SEQ ID NO: 9 with a soluble fumarate reductase TbFrd having a sequence as shown in SEQ ID NO: 1; more preferably, a mitochondrial localization signal peptide encoded by the mitochondrial localization sequence is located at the N-terminus of a fusion protein expressed by the gene mTbFrd;
preferably, the gene YlMdh2 encoding the malate dehydrogenase has a gene number of YALI0E14190p;
preferably, the gene SpMae1 encoding the dicarboxylate transport protein having a sequence as shown in SEQ ID NO: 6 is derived from *Schizosaccharomyces pombe.*

8. A method for industrial production of succinic acid, **characterized in that** the method comprises culturing and fermenting an engineered yeast strain according to any one of claims 1-5.

9. The method according to claim 8, **characterized in that** a method for culturing and fermenting the engineered yeast strain comprises: culturing and fermenting the engineered yeast strain in YPD medium;
preferably, the method for culturing and fermenting the engineered yeast strain comprises: controlling a temperature at 25-3 5°C, preferably 30°C during a fermentation phase, maintaining an aeration rate of 0.5-5 vvm, preferably 1.0 vvm, and controlling a stirring speed at 100-800 rpm, preferably 500 rpm;
preferably, the method for culturing and fermenting the engineered yeast strain further comprises monitoring a glucose concentration in a fermentation broth and supplementing glucose to 50-60 g/L when the glucose concentration falls below 10 g/L; the method does not require pH adjustment;
preferably, the YPD medium comprises: 2% tryptone, 1% yeast extract, and 6% glucose.

10. An application of an engineered yeast strain according to any one of claims 1-5 or a method for industrial production of succinic acid according to claim 8 or 9 in the fields of chemicals, biodegradable materials, food, and pharmaceuticals.
